(19) Europäisches Patentamt
European Patent Office
Office européen des brevets

(11) Veröffentlichungsnummer: **0 042 054**
**B1**

# (12) EUROPÄISCHE PATENTSCHRIFT

(45) Veröffentlichungstag der Patentschrift: **31.07.85**

(51) Int. Cl.⁴: **C 07 D 209/08**, A 61 K 31/40

(21) Anmeldenummer: **81103018.8**

(22) Anmeldetag: **21.04.81**

(54) **1-Amino-3-phenyl-indole, deren Salze und diese Indole enthaltende Arzneimittel.**

(30) Priorität: **12.06.80 DE 3022097**

(43) Veröffentlichungstag der Anmeldung: **23.12.81 Patentblatt 81/51**

(45) Bekanntmachung des Hinweises auf die Patenterteilung: **31.07.85 Patentblatt 85/31**

(84) Benannte Vertragsstaaten: **CH DE FR GB IT LI**

(56) Entgegenhaltungen:
**CH - A - 583 195**
**DE - A - 2 512 702**

**Chemical Abstracts Band 93, Nr. 13, 29 September 1980 Columbus, Ohio, USA F. SCHATZ et al. "1-Amino-3-phenylindoles with antidepressant activity. Binodaline hydrochloride and related substances" Seite 18, Spalte 2, Abstract Nr. 125412n**

**Die Akte enthält technische Angaben, die nach dem Eingang der Anmeldung eingereicht wurden und die nicht in dieser Patentschrift enthalten sind.**

(73) Patentinhaber: **SIEGFRIED AKTIENGESELLSCHAFT, CH-4800 Zofingen (CH)**

(72) Erfinder: **Zirngibl, Ludwig, Dr., Eisengrubenweg 16, CH-4800 Zofingen (CH)**
Erfinder: **Jahn, Ulrich, Dr., Kirchmoosstrasse 13, CH-4800 Zofingen (CH)**
Erfinder: **Thiele, Kurt, Dr., Rebbergstrasse 47d, CH-4800 Zofingen (CH)**

(74) Vertreter: **Jaeger, Klaus, Dr.rer.nat. Dipl.-Chem. et al,**
***Jaeger, Grams & Pontani Patentanwälte***
**Bergstrasse 48 1/2, D-8035 München-Gauting (DE)**

**EP 0 042 054 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Aus der DE 2 512 702 A1 sind Hydrazine bekannt, deren eines Stickstoffatom Ringglied des fünfgliedrigen Ringes eines (4.3.0)-Heterobicyclus bildet, während das andere mit mindestens einer organischen Gruppe substituiert ist, die mindestens zwei nicht einem Benzolring angehörende Kohlenstoffatome aufweist. In diesem Rahmen sind namentlich Derivate des 1-Amino-3-phenyl-indols als antidepressiv wirksam beschrieben, bei denen die 1-Aminogruppe entweder mit einer Acylgruppe, beispielsweise einer Acetylgruppe, oder aber mit zwei niederen Alkylgruppen substituiert ist. Im Fall der Alkylgruppensubstitution können eine oder beide Alkylgruppen jeweils nochmals eine tertiäre Aminogruppe tragen. Aus diesem engeren Substanzkreis hat sich inzwischen das Binodalin (INN), insbesondere in Form seines Hydrochlorids, als antidepressiver Wirkstoff bewährt. Dabei ist »Binodalin« die Kurzbezeichnung für 1-[N-Methyl-N-(N',N'-dimethylaminoethyl)-amino]-3-phenyl-indol (CARNO 57647-35-5).

Andere Substanzen dagegen, die in der genannten Substanzklasse mit umfaßt sind, haben aus den verschiedensten Gründen keine praktische Bedeutung als antidepressive Wirkstoffe erlangen können.

Der Erfindung liegt die Aufgabe zugrunde, neue 1-Amino-3-phenyl-indole der an sich bekannten Substanzklasse der in (4.3.0)-Heterobicyclen eingebundenen Hydrazine zu schaffen, die sich durch Abwesenheit kardiotoxischer Nebenwirkungen und rasch und mit hoher Effektivität eintretende antidepressive Wirkungen auszeichnen.

Zur Lösung dieser Aufgabe sieht die Erfindung neue 1-Amino-3-phenyl-indole der in Formel I genannten Art und deren pharmazeutisch unbedenkliche Salze vor.

Die Erfindung schafft also 1-Amino-3-phenyl-indole der Formel

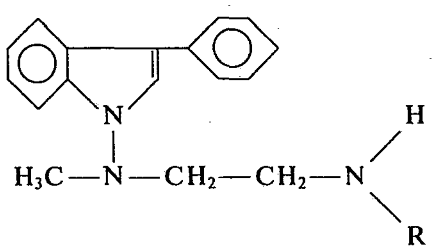

(I)

und deren pharmazeutisch unbedenklichen Salze, wobei in der Formel I R Wasserstoff, Methyl, Ethyl, n-Propyl, i-Propyl, n-Butyl, sec-Butyl oder tert-Butyl bedeutet.

Als Salze kommen insbesondere die Salze mit anorganischen Säuren, vorzugsweise die Hydrochloride, in Betracht. Von den Salzen mit organischen Säuren werden in auch präparativer Hinsicht überraschend gute Ergebnisse mit den Pikraten erhalten.

Die 1-Amino-3-phenyl-indole der Erfindung weisen sowohl in Form ihrer freien Basen als auch in Form ihrer Salze eine erstaunlich hohe antidepressive Aktivität auf und werden daher vorzugsweise als antidepressive Wirkstoffe in üblichen Arzneimittelformulierungen verwendet.

Die Herstellung der 1-Amino-3-phenyl-indole erfolgt über einzeln an sich aus analogen Verfahren bekannte Verfahrensstufen, die der herzustellenden Zielverbindung entsprechend zusammengestellt werden. Die Synthesen nehmen ihren Ausgang vorzugsweise von einem entsprechenden 1-Acetylamino-3-phenylindol der Formel

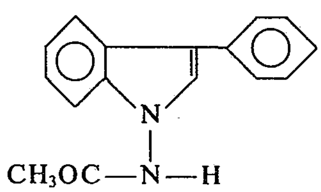

(II)

Die 1-Acetylamino-3-phenyl-indole der Formel II sind auf den verschiedensten Wegen ohne weiteres zugänglich (vgl. beispielsweise J. chem. Soc. 1964, 4037).

Das 1-Acetylaminoindol der Formel II kann durch Umsetzen mit Methyl-p-Toluolsulfonat in Gegenwart von Natriumhydrid über das so erhaltene 1-Acetylmethylaminoindol, das anschließend mit KOH verseift wird, in das entsprechende 1-Methylamino-3-phenyl-indol überführt werden. Durch Umsetzen mit den entsprechenden N-Benzyl-N-alkylaminoethylhalogeniden und Hydrogenolyse der erhaltenen Benzylderivate ist dann das 1-Methylamino-3-phenyl-indol in das Zielprodukt überführbar.

Die Erfindung ist im folgenden anhand eines Ausführungsbeispiels näher erläutert.

Beispiel

1-[N-Methyl-N-(N'-methylaminoethyl)-amino]-3-phenyl-indol

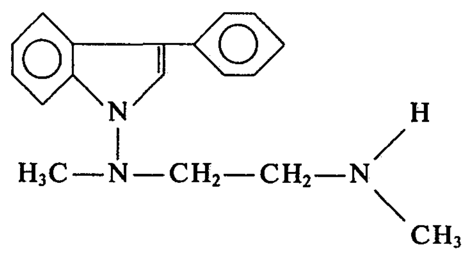

Ein mit einem Rückflußkühler und einem Rührer ausgerüsteter Dreihalskolben wird mit 8,5 g einer 15%igen Suspension von $NaNH_2$ in Toluol (ca. 110 mmol) beschickt. Zu dieser Suspension, die auf 5° C abgekühlt ist, wird unter ständigem Rühren eine Lösung von 22,2 g (100 mmol) 1-Methylamino-3-phenyl-indol in 200 ml Toluol eingetropft. Nach Abschluß der Zugabe wird weitere 3 h bei 5° C gerührt.

Parallel dazu werden 33 g (150 mmol) 2-(Benzyl-methyl-amino)-ethylchloridhydrochlorid in Eiswasser gelöst und mit Toluol versetzt. Das Reaktionsgemisch wird bis zur basischen Reaktion mit NaOH versetzt. Anschließend wird 15 min gerührt. Nach dem Trennen der flüssigen Phasen wird die wäßrige Schicht mit Toluol extrahiert. Die kombinierten organischen Phasen werden über Magnesiumsulfat und Calciumsulfat getrocknet. Anschließend wird filtriert.

Die so erhaltene getrocknete und filtrierte toluolische Lösung des Aminoethylchlorids wird unter weiterhin ständigem Rühren und Kühlen des Reaktionsgemisches auf 5° C im Verlauf von 1 h in das Indolreaktionsgemisch eingetropft. Nach Abschluß der Zugabe wird weitere 3 h bei einer Temperatur von 5 bis 10° C gerührt. Das Reaktionsgemisch wird danach bei dieser Temperatur weitere 16 h stehengelassen. Dann wird zunächst zweimal mit Wasser und anschließend zweimal mit je 200 ml 2n HCl gewaschen. Das Reaktionsgemisch trennt sich beim Stehenlassen in drei Schichten. Die oberste Schicht besteht im wesentlichen nur aus Toluol und wird verworfen. Die mittlere wäßrige Schicht und die untere ölige Schicht werden vereint und mit Toluol ausgeschüttelt. Nach erneutem Trennen der Phasen und Verwerfen der Toluolphase wird mit Chloroform extrahiert. Die nach der Extraktion erhaltene Chloroformphase wird mit wäßriger 1n HC1 gewaschen, um Reste von nicht umgesetzten basischen Ausgangsmaterial zu entfernen. Danach wird die Chloroformschicht mit 2n NaOH gewaschen, getrocknet und schließlich unter vermindertem Druck eingedampft. Als Rückstand wird ein Öl erhalten, das aus 60 ml Isopropanol auskristallisiert. Nach Umkristallisieren aus Isopropanol werden schließlich 17 g reines kristallines 1-[N-Methyl-N-(N'-benzyl-N'-methyl-aminoethyl)-amino]-3-phenyl-indol mit einem Schmelzpunkt von 73 bis 73,5° C erhalten. Die Identität des erhaltenen Produkts wird durch die Elementaranalyse und spektrographisch bestätigt.

44,4 g (120 mmol) des so hergestellten 1-[N-Methyl-N-(N'-benzyl-N'-methyl-aminoethyl)-amino]-3-phenyl-indols werden in einem Gemisch von 320 ml 95%igem wäßrigem Ethanol und 65 ml Eisessig gelöst und in Gegenwart von 5,8 g Palladiumkohle (5% Pd) unter Schütteln 15 h hydriert. Das Reaktionsgemisch wird filtriert und unter vermindertem Druck eingedampft. Der Rückstand wird in Wasser aufgenommen, alkalisch gemacht und mit Methylenchlorid extrahiert. Nach dem Trocknen und Abziehen des Methylenchlorids wird als Extrakt ein Öl erhalten, das unter Verwendung von Chloroform auf einer mit Kieselsäure gefüllten Säule chromatographiert wird. Die durch Dünnschichtchromatographie als rein ermittelten Fraktionen werden vereinigt und eingedampft. Dabei werden 16 g reines Produkt in Form eines hellen Öls erhalten. Die Idendität des Zielproduktes wird durch die Elementaranalyse sowie spektrographisch bestätigt.

In analoger Weise werden durch katalytische Debenzylierung (Hydrogenolyse) der analog hergestellten entsprechenden N'-Benzylverbindung die folgenden Substanzen hergestellt:

1-[N-Methyl-N-(N'-ethylaminoethyl)-amino]-3-phenyl-indol;
1-[N-Methyl-N-(N'-isopropylaminoethyl)-amino]-3-phenyl-indol und
1-[N-Methyl-N-(N'-butylaminoethyl)-amino]-3-phenyl-indol.

In entsprechender Weise sind auch das isomere N'-n-Propylderivat und die beiden anderen isomeren N'-Butylderivate zugänglich.

**Patentansprüche**

1. 1-Amino-3-phenyl-indole der Formel

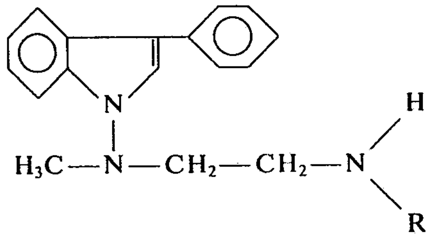

(I)

und deren pharmazeutisch unbedenkliche Salze, wobei in der Formel I R Wasserstoff, Methyl, Ethyl, n-Propyl, i-Propyl, n-Butyl, sec-Butyl und tert-Butyl bedeutet.

2. Verbindungen nach Anspruch 1 zur Verwendung als antidepressive Wirkstoffe in humanmedizinischen Arzneimittelformulierungen.

3. Arzneimittel, enthaltend mindestens eine der Basen nach Anspruch 1 und/oder mindestens eines ihrer pharmazeutisch unbedenklichen Salze als Wirkstoff.

**Claims**

1-amino-3-phenylindoles according to formula

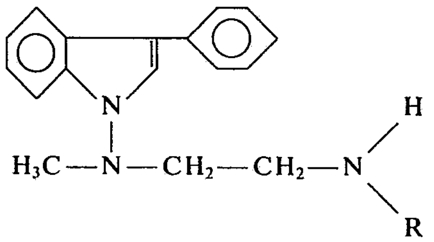

(I)

and the pharmaceutically acceptable salts thereof, wherein R is hydrogen, methyl, ethyl, n-propyl, isopropyl, n-butyl, sec-butyl and tert-butyl.

2. Compounds according to claim 1 for application as antidepressant active substances in human medical drug formulations.

3. Drugs, containing at least one of the bases according to claim 1 and/or at least one of the pharmaceutically acceptable salts of said bases as active substance.

**Revendications**

1-Amino-1 phényl-3 indoles répondant à la formule I:

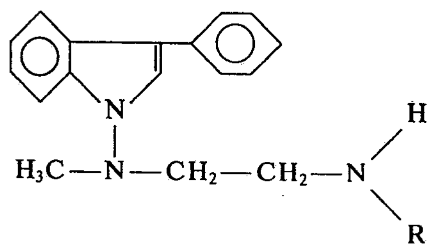

(I)

dans laquelle R représente l'hydrogène ou un radical méthyle, éthyle, n-propyle, isopropyle, n-butyle, sec-butyle ou tert-butyle, et leurs sels acceptables du point de vue pharmaceutique.

2. Composés selon la revendication 1 pour leur application en tant que substances anti-dépressives dans des formulations médicamenteuses destinées à la médecine humaine.

3. Médicament contenant, comme substance active, au moins une des bases selon la revendication 1 et/ou au moins un de leurs sels acceptables du point de vue pharmaceutique.